# EUROPEAN PATENT APPLICATION

(11) **EP 4 822 034 A1**
(43) Date of publication of application: **14.10.2026**
(21) Application number: 25769176.6
(22) Date of filing: 10.02.2025
(51) Int. Cl.: C12Q 1/6844, C12Q 1/6886

(54) **PRIMER-PROBE COMBINATION FOR EARLY DETECTION OF ENDOMETRIAL CANCER, METHYLATION DETECTION KIT, AND USE THEREOF**

(30) Priority: 15.03.2024 CN 202410299405
(71) Applicant: Sun Yat-Sen University Cancer Center, Guangzhou, Guangdong 510000 (CN)
(72) Inventor: JIA, Weihua, Guangzhou, Guangdong 510000 (CN); LIAO, Ying, Guangzhou, Guangdong 510000 (CN); DU, Yan, Guangzhou, Guangdong 510000 (CN); MENG, Yifan, Guangzhou, Guangdong 510000 (CN)
(74) Representative: Chung, Hoi Kan
(86) International application number: PCT/CN2025/076646
(87) International publication number: WO 2025/190002

(57) **Abstract**

The present disclosure relates to a primer-probe combination for early detection of endometrial cancer, a methylation detection kit, and the use thereof. Provided in the present invention is a methylation gene marker for endometrial cancer detection, which specifically comprises at least one of VSX1, SYT1, ZNF132 and VWC2 genes. Measuring the methylation levels of the genes can remarkably distinguish subjects with endometrial cancer from subjects without cancer. The gene marker of the present invention can well identify subjects with endometrial cancer and subjects without cancer from each of tissue/urine/brush smear/swab/blood/lavage fluid specimens, and can achieve a good detection effect simply by means of a single index and further improve the detection efficiency by means of multi-index combination.

## Description

### TECHNICAL FIELD

The present disclosure belongs to the field of biomedical technology, and particularly relates to a primer-probe combination for early detection of endometrial cancer, a methylation detection kit, and use thereof.

### BACKGROUND

Endometrial cancer is a common epithelial malignant tumor occurring in the endometrium, accounting for about 20%-30% of gynecological malignancies. Endometrial cancer is more common in perimenopausal and postmenopausal women, with a high incidence age of 50-70 years and a median diagnosis age of 61 years. In recent years, with the increasing prevalence of risk factors for endometrial cancer, especially obesity and population aging, the incidence of endometrial cancer has gradually risen. Additionally, there is a trend of younger onset of endometrial cancer, with a significant increase in incidence among premenopausal women. The treatment efficacy of endometrial cancer is closely related to the clinical stage, and patients with early-stage endometrial cancer usually have a better prognosis. According to statistics, the five-year survival rate for early-stage (Stage I, II) patients can exceed 70%, for Stage III patients it is about 40-50%, while for Stage IV patients it is only 15-20%. Early diagnosis is of great significance for endometrial cancer.

The initial symptom for most endometrial cancer patients is irregular vaginal bleeding or vaginal discharge. Since this symptom is not specific, approximately 14% of women of reproductive age and 4%-11% of postmenopausal women experience it, with only 0.33%-1.04% ultimately diagnosed with endometrial cancer. Based on this symptom, extensive clinical examinations are often required after consultation to confirm whether endometrial cancer is present, including but not limited to imaging tests (such as transvaginal ultrasound), laboratory tests (detecting common clinical markers like CA125 and HE4), and endometrial biopsy (diagnostic curettage and hysteroscopic curettage, which remove endometrial tissue for further histopathological examination). Imaging and laboratory tests have low sensitivity and specificity, while endometrial biopsy is an invasive procedure that easily causes physical discomfort. Therefore, there is an urgent need to develop a convenient, rapid, and non-invasive detection method that can accurately identify endometrial cancer at an early stage.

DNA methylation is a type of epigenetic modification, involving the addition of a methyl group to the 5-carbon atom of a cytosine base; this modification is typically associated with gene silencing. DNA methylation is a key epigenetic regulator of gene expression, often leading to defects in gene expression. Increased methylation of tumor suppressor genes is an early event in many tumors. Methylation signals are widely recognized as a source of tumor screening markers, offering advantages such as early appearance during tumorigenesis, stable signals, and convenient detection methods. However, DNA methylation has not yet achieved effective clinical application in the early screening and diagnosis of endometrial cancer.

### SUMMARY

To overcome the shortcomings and deficiencies of the prior art described above, the primary object of the present disclosure is to provide a methylated gene marker for the early detection of endometrial cancer.

Through screening, the present disclosure has obtained specific methylation early screening markers for endometrial cancer, including at least one of the VSX1, SYT1, ZNF132, and VWC2 genes. Detecting the methylation of these genes distinguishes endometrial cancer from non-cancer individuals, and the stability and effectiveness of the markers of the present disclosure have been validated by testing in various non-invasively collected sample types.

Another object of the present disclosure is to provide a detection kit for diagnosing endometrial cancer.

The present disclosure has identified sites that can accurately distinguish endometrial cancer from other diseases/healthy individuals by eliminating background signal interference in various non-invasively collected samples, and has established a stable and reliable marker detection kit based on them.

A further object of the present disclosure is to provide a use of the aforementioned kit in preparation of a product for diagnosis of endometrial cancer.

The objects of the present disclosure are achieved through the following solutions:
According to a first aspect of the present disclosure, the present disclosure provides a methylated gene marker for endometrial cancer detection. The methylated gene marker specifically includes at least one of the VSX1, SYT1, ZNF132, and VWC2 genes. The detection refers to diagnosing endometrial cancer by detecting the methylation level of at least one of the target genes VSX1, SYT1, ZNF132, and VWC2.

According to a second aspect of the present disclosure, the present disclosure provides a methylated gene marker for endometrial cancer detection. The methylated gene marker includes a methylated nucleic acid sequence in at least one target region of at least one of the target genes VSX1, VWC2, ZNF132, and SYT1, the target region selected from the methylation of at least one of the following segments in at least one of the VSX1, VWC2, ZNF132, and SYT1 genes:
VSX1 gene: Chr20: 25058334-25058603; Chr20: 25061937-25062310 or Chr20: 25061874-25062088; Chr20: 25062398-25062760 or Chr20: 25062376-25062484; Chr20: 25062681-25062788 or Chr20: 25062655-25062739; Chr20: 25062736-25062940 or Chr20: 25062749-25062900;
SYT1 gene: Chr12: 79258369-79258486 or Chr12: 79258392-79258498;
ZNF132 gene: Chr19: 58951204-58951524 or Chr19: 58951402-58951524; Chr19: 58951554-58951637; Chr19: 58951672-58952006 or Chr19: 58951724-58951825;
VWC2 gene: Chr7: 49812992-49813086; Chr7: 49813051-49813343 or Chr7: 49813066-49813161; Chr7: 49813455-49813811 or Chr7: 49813387-49813537; Chr7: 49814721-49815073 or Chr7: 49814765-49814936; Chr7: 49815144-49815236; Chr7: 49815290-49815635 or Chr7: 49815340-49815439.

The genes described in this disclosure refer to the human genome version GRCh37 (hg19) as the reference genome.

This disclosure has identified early screening markers specific for endometrial cancer methylation through screening, including at least one gene among VSX1, SYT1, ZNF132, and VWC2. Detecting the methylation levels of these genes can significantly distinguish endometrial cancer from non-cancer individuals. The stability and effectiveness of these markers have been validated by testing in various non-invasive sample types. This disclosure provides new markers and diagnostic strategies for the diagnosis of endometrial cancer and its precancerous lesions.

Furthermore, diagnosis of endometrial cancer can be achieved by detecting any at least one methylation region corresponding to any at least one target gene in vitro samples. The samples can be any body fluid, exfoliated cells, or tissue samples, such as body fluid samples including urine and blood, and exfoliated cell collection samples like swabs, brushings, and lavage fluid. The gene markers of this disclosure can effectively distinguish endometrial cancer from non-cancer individuals in tissue/urine/brushing/swab samples, with single indicators achieving good detection results, and combined multiple indicators further improving detection performance.

According to a third aspect of the present disclosure, a detection primer for endometrial cancer detection is provided for detecting the methylation status of a gene methylation region of the aforementioned marker, with the nucleotide sequences of the detection primers being at least one of the following:
VSX1 gene:
   Chr20: 25058334-25058603 corresponds to detection primers SEQ ID NO:1-2;
   Chr20: 25061937-25062310 corresponds to detection primers SEQ ID NO:4-5;
   Chr20: 25061874-25062088 corresponds to detection primers SEQ ID NO:6-7;
   Chr20: 25062398-25062760 corresponds to detection primers SEQ ID NO:9-10;
   Chr20: 25062376-25062484 corresponds to detection primers SEQ ID NO:12-13;
   Chr20: 25062681-25062788 corresponds to detection primers SEQ ID NO:15-16;
   Chr20: 25062655-25062739 corresponds to detection primers SEQ ID NO:18-19;
   Chr20: 25062736-25062940 corresponds to detection primers SEQ ID NO:21-22;
   Chr20: 25062749-25062900 corresponds to detection primers SEQ ID NO:23-24;
SYT1 gene:
   Chr12: 79258369-79258486 corresponds to detection primers SEQ ID NO:26-27;
   Chr12: 79258392-79258498 corresponds to detection primers SEQ ID NO:29-30;
ZNF132 gene:
   Chr19: 58951204-58951524 corresponds to detection primers SEQ ID NO:32, SEQ ID NO:34;
   Chr19: 58951402-58951524 corresponds to detection primers SEQ ID NO:33, SEQ ID NO:34;
   Chr19: 58951554-58951637 corresponds to detection primers SEQ ID NO:36-37;
   Chr19: 58951672-58952006 corresponds to detection primers SEQ ID NO:39-40;
   Chr19: 58951724-58951825 corresponds to detection primers SEQ ID NO:41-42;
VWC2 gene:
   Chr7: 49812992-49813086 corresponds to detection primers SEQ ID NO:44-45;
   Chr7: 49813051-49813343 corresponds to detection primers SEQ ID NO:47-48;
   Chr7: 49813066-49813161 corresponds to detection primers SEQ ID NO:49-50;
   Chr7: 49813455-49813811 corresponds to detection primers SEQ ID NO:52-53;
   Chr7: 49813387-49813537 corresponds to detection primers SEQ ID NO:54-55;
   Chr7: 49814721-49815073 corresponds to detection primers SEQ ID NO:57-58;
   Chr7: 49814765-49814936 corresponds to detection primers SEQ ID NO:59-60;
   Chr7: 49815144-49815236 corresponds to detection primers SEQ ID NO:62-63;
   Chr7: 49815290-49815635 corresponds to detection primers SEQ ID NO:65-66;
   Chr7: 49815340-49815439 corresponds to detection primers SEQ ID NO:67-68.

According to a fourth aspect of the present disclosure, a detection probe for early screening and diagnosis of endometrial cancer is provided for detecting the methylation status of a gene methylation region of the aforementioned marker, the nucleotide sequence of the probe being one of the following:
VSX1 gene:
   Chr20: 25058334-25058603 corresponds to detection probe SEQ ID NO:3;
   Chr20: 25061937-25062310 corresponds to detection probe SEQ ID NO:8;
   Chr20: 25061874-25062088 corresponds to detection probe SEQ ID NO:8;
   Chr20: 25062398-25062760 corresponds to detection probe SEQ ID NO:11;
   Chr20: 25062376-25062484 corresponds to detection probe SEQ ID NO:14;
   Chr20: 25062681-25062788 corresponds to detection probe SEQ ID NO:17;
   Chr20: 25062655-25062739 corresponds to detection probe SEQ ID NO:20;
   Chr20: 25062736-25062940 corresponds to detection probe SEQ ID NO:25;
   Chr20: 25062749-25062900 corresponds to detection probe SEQ ID NO:25;
SYT1 gene:
   Chr12: 79258369-79258486 corresponds to detection probe SEQ ID NO:28;
   Chr12: 79258392-79258498 corresponds to detection probe SEQ ID NO:31;
ZNF132 gene:
   Chr19: 58951204-58951524 corresponds to detection probe SEQ ID NO:35;
   Chr19: 58951402-58951524 corresponds to detection probe SEQ ID NO:35;
   Chr19: 58951554-58951637 corresponds to detection probe SEQ ID NO:38;
   Chr19: 58951672-58952006 corresponds to detection probe SEQ ID NO:43;
   Chr19: 58951724-58951825 corresponds to detection probe SEQ ID NO:43;
VWC2 gene:
   Chr7: 49812992-49813086 corresponds to detection probe SEQ ID NO:46;
   Chr7: 49813051-49813343 corresponds to detection probe SEQ ID NO:51;
   Chr7: 49813066-49813161 corresponds to detection probe SEQ ID NO:51;
   Chr7: 49813455-49813811 corresponds to detection probe SEQ ID NO:56;
   Chr7: 49813387-49813537 corresponds to detection probe SEQ ID NO:56;
   Chr7: 49814721-49815073 corresponds to detection probe SEQ ID NO:61;
   Chr7: 49814765-49814936 corresponds to detection probe SEQ ID NO:61;
   Chr7: 49815144-49815236 corresponds to detection probe SEQ ID NO:64;
   Chr7: 49815290-49815635 corresponds to detection probe SEQ ID NO:69;
   Chr7: 49815340-49815439 corresponds to detection probe SEQ ID NO:69.

According to a fifth aspect of the present disclosure, the present disclosure provides a use of a reagent for detecting methylation of a target gene in preparation of a detection kit or device, wherein the detection kit or device is used for detecting, screening, or diagnosing endometrial cancer; the target gene is selected from at least one of the following genes: VSX1, SYT1, ZNF132, VWC2.

The reagent includes at least one of an antibody, a probe, a primer, and a mass spectrometry detection reagent specifically targeting the detection of the target gene. Specifically, the reagent includes a product for specifically detecting the target gene, which can be at least any one of an antibody, a probe, a primer, and a mass spectrometry detection reagent; wherein the primer includes the detection primer according to claim 3, and may also be other reagents with similar functions. Additionally, the kit may take the form of kits similar to existing products, and the device may be sequence detection devices. Either primers or probes may be selected individually or used in combination as needed.

More specifically, the primer includes at least one of the above detection primers for endometrial cancer detection.

More specifically, the probe includes at least one of the above detection probes for early screening and diagnosis of endometrial cancer.

Furthermore, the detection sample for the detection kit or device may be any body fluid, exfoliated cells, tissue samples, etc. For example, including body fluid samples such as urine and blood, and exfoliated cell collection samples such as swabs, brushings, and lavage fluid.

According to a sixth aspect of the present disclosure, the present disclosure provides a kit for diagnosing endometrial cancer, including a reagent for detecting methylation of a target gene; the target gene is selected from at least one of the following genes: VSX1, SYT1, ZNF132, VWC2.

The reagent includes at least one of an antibody, a probe, a primer, and a mass spectrometry detection reagent specifically targeting the detection of the target gene, wherein the primer includes at least one of the above detection primers for endometrial cancer detection. The probe includes at least one of the above detection probes for early screening and diagnosis of endometrial cancer.

Furthermore, the aforementioned reagents can detect the methylation level of the target gene by at least one of the following methods: quantitative fluorescent methylation-specific PCR, methylation-specific PCR, whole-genome methylation sequencing, digital PCR, bisulfite sequencing, pyrosequencing, methylation-specific high-resolution melting analysis, methylation-specific microarray, methylation-specific high-performance liquid chromatography, methylation-sensitive restriction enzyme digestion or quantitative fluorescence, reduced representation bisulfite sequencing, matrix-assisted laser desorption/ionization time-of-flight mass spectrometry, etc.

It should be noted that methods for detecting gene methylation are well-known to those skilled in the art, including but not limited to the methods listed above; all of these methods can achieve the detection of methylation levels in the target gene. Based on this, reagents suitable for the aforementioned methods can all be prepared into kits for the diagnosis of endometrial cancer.

Furthermore, the kit further includes a primer pair and a probe for a reference gene, wherein the reference gene is the ACTB gene. The nucleotide sequence of the primer pair for the reference gene is as follows, and the nucleotide sequence of the probe is as follows:
Forward primer: 5'-TGGTGATGGAGGAGGTTTAGTAAGT -3' (SEQ ID NO: 70)
Reverse primer: 5'-AACCAATAAAACCTACTCCTCCCTTAA -3' (SEQ ID NO: 71)
Probe: 5'-ACCACCACCCAACACACAATAACAAACACA-3' (SEQ ID NO: 72).

Furthermore, the 5' reporter fluorophores for the probes specific to the target region and the reference gene are one or more of FAM, VIC, HEX, NED, ROX, Red, TET, Texas, JOE, TAMRA, CY3, CY5; the 3' quencher fluorophores for the probes specific to the target region and the reference gene are one or more of MGB, BHQ-1, BHQ-2, BHQ-3, IBRQ, MGB-NFQ, DABCYL, ECLIPSE.

Furthermore, the kit further includes a negative control, a positive control, and a no-template control. The no-template control is ddH₂O. The positive control is plasmid DNA with fully methylated target regions. The negative control is plasmid DNA with completely unmethylated target regions.

The present disclosure also proposes the use of the aforementioned gene markers in preparation of products for diagnosing endometrial cancer.

The present disclosure also proposes the use of the aforementioned kit in preparation of products for diagnosing endometrial cancer.

Furthermore, the diagnostic product may include any one of a kit, a preparation, and a chip. The gene markers of the present disclosure can be used to prepare various types of diagnostic products, which can be flexibly selected based on actual circumstances.

The present disclosure obtains endometrial cancer-specific methylation early screening markers through screening, including at least one gene among YSX1, SYT1, ZNF132, and VWC2. Detecting the methylation levels of these genes can significantly distinguish endometrial cancer from non-cancerous individuals; by detecting any at least one methylation region corresponding to any at least one target gene in an in vitro sample, diagnosis of endometrial cancer can be achieved. The gene markers of the present disclosure can effectively differentiate endometrial cancer from non-cancerous individuals in samples such as tissue/urine/brush smears/swabs/blood/lavage fluid, with a single indicator achieving good detection performance, and multiple indicators combined further enhancing detection efficacy.

### BRIEF DESCRIPTION OF DRAWINGS

To more clearly illustrate the technical solutions of the embodiments of the present disclosure, the following briefly introduces the drawings required for the embodiments. It should be understood that the following drawings only show some embodiments of the present disclosure and therefore should not be considered as limiting the scope. For those of ordinary skill in the art, other related drawings can be obtained based on these drawings without creative effort.
FIG. 1 is a schematic diagram of the process for screening and constructing the system of endometrial cancer gene markers in the present disclosure.
FIGS. 2-3 show the methylation levels of the gene markers in the present disclosure detected by real-time fluorescent quantitative PCR amplification.
FIG. 4 shows the M-index distribution of methylation levels of the four gene markers in urine samples in the present disclosure.
FIG. 5 shows the M-index distribution of methylation levels of the four gene markers in multicenter urine samples in the present disclosure.
FIG. 6 shows the M-index distribution of methylation levels of the four gene markers in multicenter urine samples from patients with endometrial cancer and benign gynecological diseases in the present disclosure.
FIG. 7 shows the M-index distribution of methylation levels of the four gene markers in cervical brush smear samples in the present disclosure.
FIG. 8 shows the M-index distribution of methylation levels of the four gene markers in vaginal swab samples in the present disclosure.

### DETAILED DESCRIPTION OF THE EMBODIMENTS

The present disclosure is further described in detail below with reference to embodiments, but the implementation of the present disclosure is not limited thereto. Materials involved in the following embodiments can be obtained commercially unless otherwise specified. The methods described are conventional unless otherwise stated.

This disclosure utilizes the endometrial cancer 450k open data from The Cancer Genome Atlas (TCGA) database (http://cancergenome.nih.gov/). Through data modeling and analysis, methylation sites strongly correlated with the occurrence of endometrial cancer were identified, and multiple regions of four genes-VSX1, VWC2, ZNF132, and SYT1-were selected as having potential as DNA methylation biomarkers for detecting endometrial cancer. The detection method of this disclosure was applied to test these methylation sites in cancer tissues and adjacent non-cancerous tissues from endometrial cancer patients, urine samples from endometrial cancer patients (182 cases) and non-cancer individuals (334 cases), cervical brush samples from endometrial cancer patients and healthy individuals, and vaginal swab samples from endometrial cancer patients and healthy individuals. Using primer-probe combinations designed for multiple regions of the aforementioned four genes, methylation signals in DNA from cancer tissues/urine/cervical brush/vaginal swab samples of endometrial cancer individuals were significantly higher than in non-cancer individuals, indicating that the methylation levels at these sites can sensitively and specifically reflect the occurrence of endometrial cancer. Furthermore, the urine samples collected in this disclosure included patients with benign gynecological diseases (such as uterine fibroids, ovarian chocolate cysts, uterine polyps, endometritis, etc.), and the aforementioned sites could also distinguish these benign gynecological disease patients from endometrial cancer patients. This demonstrates that the combination of methylation sites selected in this disclosure exhibits high endometrial cancer-related signals in urine DNA and performs well in differentiating endometrial cancer from benign tumors requiring differential diagnosis, showing superior sensitivity for detecting endometrial cancer. Meanwhile, using urine as a test sample is non-invasive, significantly reducing patient burden and increasing compliance with testing. Therefore, the methylated gene markers of the four genes in this disclosure can serve as non-invasive detection markers for early diagnosis of endometrial cancer.

### Example 1: Screening of Methylated Gene Markers for Endometrial Cancer

To identify DNA methylation markers that can characteristically distinguish endometrial cancer patients from non-cancer groups, this disclosure first obtained 450K methylation chip data of 425 cancer tissues and 34 adjacent non-cancerous tissues of endometrial cancer from the TCGA public database, screening for sites significantly hypermethylated in endometrial cancer tissues. After data quality control, 385,577 methylation sites were included in the analysis. Further using the CHAMP.DMP method for differential site screening, with the criteria of FDR-adjusted *P* value <0.05 and average methylation rate in cancer tissues minus average methylation rate in adjacent non-cancerous tissues >0.35, a total of 8,730 differential sites were screened.

Since sampling methods such as urine, cervical brush, vaginal swab, and blood inevitably include components like leukocytes or urogenital epithelial cells in addition to tumor-derived cells or DNA. The incorporation of these non-target exogenous DNAs may interfere with the detection of target markers as background signals. Therefore, careful handling of background signal interference is required when screening markers. Considering the above, this disclosure further incorporated 450K methylation chip data from 78 female urogenital adjacent non-cancerous tissues and 338 leukocytes in the TCGA and GEO databases, performing CHAMP.DMP differential tests with the data from 425 endometrial cancer tissues. Sites meeting all the following conditions were included in the screening scope: (1) FDR-adjusted *P* value <0.05 in both differential tests; (2) average methylation rate in cancer tissues minus average methylation rate in urogenital adjacent non-cancerous tissues >0; (3) average methylation rate in cancer tissues minus average methylation rate in leukocytes >0; (4) average methylation rate in urogenital adjacent non-cancerous tissues <0.1; and (5) average methylation rate in leukocytes <0.1. Intersecting the sites meeting these requirements with the differential sites from the endometrial cancer/adjacent differential screening resulted in 1,464 sites being included in the next screening step.

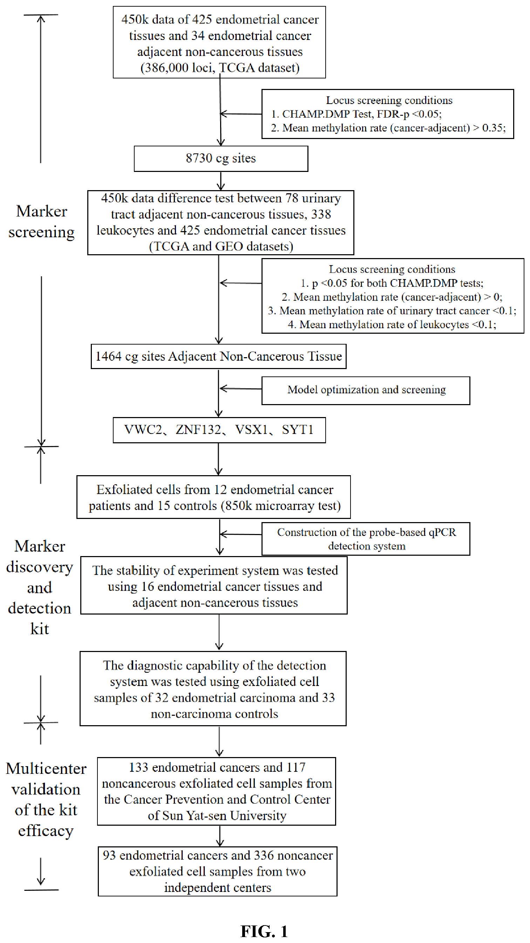

To further evaluate the methylation levels of the aforementioned 1,464 candidate sites in actual samples, this disclosure performed Illumina Epic 850K array detection on exfoliated cell samples from 12 endometrial cancer patients and 15 controls collected at Sun Yat-sen University Cancer Center and Foshan First People's Hospital. This array is an upgraded version of the 450K array, retaining most of the 450K sites while adding nearly 400,000 new methylation sites. Ultimately, this disclosure selected four genes, including VSX1, SYT1, ZNF132, and VWC2, as detection genes for endometrial cancer diagnosis. The screening process is shown in FIG. 1. The methylation sites with the best diagnostic performance for endometrial cancer for the above genes and their performance information are listed in Table 1.

The area under the curve for the optimal sites of the four genes VSX1, SYT1, ZNF132, and VWC2 ranges from 0.906 to 0.972. Among them, VWC2 and ZNF132 have AUCs greater than 0.95, demonstrating excellent diagnostic performance; VSX1 and SYT1 have AUCs above 0.90, also showing very good diagnostic performance. Different genes exhibit distinct advantages in detection sensitivity and specificity, with VWC2 and ZNF132 genes achieving specificity up to 100%, and the VSX1 gene achieving specificity up to 91.7%. The above genes demonstrate excellent discriminative ability between endometrial cancer and controls. Furthermore, sites with strong discriminative ability cluster around the optimal site; within 500bp upstream and downstream of the best site, the above genes have multiple sites with AUCs greater than 0.8, specifically VWC2, ZNF132, VSX1, and SYT1 have 8, 7, 5, and 2 sites with AUCs above 0.8, respectively. The clustering of multiple sites further confirms the certainty of using methylation of these genes as diagnostic markers for endometrial cancer and facilitates the development of methylation site detection methods. These results indicate that the four genes selected by this disclosure possess excellent discriminative ability between endometrial cancer and control populations.

**Table 1**

| Gene | A | B (hg19) | Sensitivity (%) | Specificity (%) | AUC | C |
|---|---|---|---|---|---|---|
| VWC2 | cg18206027 | CHR7: 49813486 | 83.3 | 100.0 | 0.972 | 8 |
| ZNF132 | cg24366702 | CHR19: 58951778 | 83.3 | 100.0 | 0.950 | 7 |
| VSX1 | cg14763548 | CHR20: 25062447 | 91.7 | 80.0 | 0.911 | 5 |
| SYT1 | cg03205584 | CHR12: 79258407 | 75.0 | 93.3 | 0.906 | 2 |

Note: A is the methylation site with the best predictive performance; B is the location of the best site; C is the number of sites within 500bp upstream and downstream of the best site with AUC>0.8.

### Example 2: Construction of the Endometrial Cancer Detection Method System

1. Selection of detection regions: the sequences of regions containing methylation sites for endometrial cancer diagnosis are shown in Table 2:

**Table 2**

| Genes | Region range | Region sequence(5'-3') |
|---|---|---|
| VSX1-Region 1 | chr20:250583 34-25058603 (VSX1_1_1) | CGCGCAGGAGCCCAGCAGGCCGCCCTCGGCGGAGTTGAGCACGGAG TCTGGCAGCGGGATGCAGTGGCGCACCATGGCCCCGTACAGCCCGTA CTCGGCCATCACGCTGCTGCCGCCCCAGCGCTTCTCCCGCTTGCGCCA TTTGGCCCTGCGGTTTTGAAACCAGACCTGGTTGGAGGCAGGAGAAG CAGAAGGCACACAGAAGAGACAGTGAAGAGGGGCGCCTGGAGGAG CGGAGGCGGCGTCCCAGGGCTCGGATCTTCTCTCCCG |
| VSX1-Region 2 | chr20:250619 37-25062310 (VSX1_2_1) | CGGGCCTTCCCGACCCACGCAGCTGCCAGAGGCGGAGACTGGCGCCT GGCGCTGGGGGAAAAATGGCCCTCTGGAGATGGTTGTTGAAGACCC CAGGGTCTCATCAAAAGGTTCCACCCGGAACCAGGTGGTGGCGGTCT CACATCGCTGAGGGAACAGGACCCCGGATGAGAGGCAGGGATTTAGG ATGCAGCAAGGGGCAGGCGGCGCAGCTCCGCGAATGCCCCTCCCGCG ACGGGGCCTCGCTCTGGGCTCCCCGCCTAGATCTGGGCCGCTGGGGC CAGGGGTGCGGTGGGGCGATGGTCTGTGACCCCTGCGCGGCTCAGAG CCTAGGGGACAGGGGCAGGAGCGGAAAGCGCGGGCCTGATTACCG |
| | chr20:250618 74-25062088* (VSX1_2_2) | ATCATTTCCGCCCGCGTTTCCACCTGGTACCTTGTTCGGTGCTGCTGGT GACCACCACAGGCCCGGGCCTTCCCGACCCACGCAGCTGCCAGAGG CGGAGACTGGCGCCTGGCGCTGGGGGGAAAAATGGCCCTCTGGAGAT GGTTGTTGAAGACCCCAGGGTCTCATCAAAAGGTTCCACCCGGAACC AGGTGGTGGCGGTCTCACATCGCTGA |
| VSX1-Region 3 | chr20:250623 98-25062760 (VSX1_3_1) | CTGGGCGGCAGGAACGGCACGTCCGCTAGGAGCAGGCAGGGTGCTC GAGCGGCCGCCGGCGGCTGCGTGCCGAAGCCACAGAGGAGGCCGAG TCCCAGCGGTAGGGCCCCACGCGCCAGGCTGGAGCCGTCAAGCCCCG GGCCCGGGCACGGCGCGACTGCCGGACCCTCGCAGCCAGATCCCTGT CCTGGGCCAGCGGGCGCCGGCAGCTCGGCCTCCAAGCCCAGCAGGT CCGTGATGGCGAAGCCCCGGGGGCGCGAGCCCCTAGGGGAACCGCC AGGCACCAGCGCCCTGCTGCTAGTGCGCCCGTCGGAAAGCGAGTCCC GGCCGGTCATGGTTCCTTAGCAAGCAAGGCGCGAGCCT |
| | chr20:250623 76-25062484* (VSX1_3_2) | CGGGGCAGCGGGCTCGGGGCCCCTGGGCGGCAGGAACGGCACGTCC GCTAGGAGCAGGCAGGGTGCTCGAGCGGCCGCCGGCGGCTGCGTGC CGAAGCCACAGAGGAGG |
| VSX1-Region 4 | chr20:250626 81-25062788 (VSX1_4_1) | CCAGCGCCCTGCTGCTAGTGCGCCCGTCGGAAAGCGAGTCCCGGCCG GTCATGGTTCCTTAGCAAGCAAGGCGCGAGCCTCTCTGGATCCCGTTT GCGGAGGGCCCAG |
| | chr20:250626 55-25062739* (VSX1_4_2) | CGAGCCCCTAGGGGAACCGCCAGGCACCAGCGCCCTGCTGCTAGTGC GCCCGTCGGAAAGCGAGTCCCGGCCGGTCATGGTTCCT |
| VSX1-Region 5 | chr20:250627 36-25062940 (VSX1_5_1) | TCCTTAGCAAGCAAGGCGCGAGCCTCTCTGGATCCCGTTTGCGGAGG GCCCAGCTTAGAGGAAGCTTTATAGGATTGGGCTGCCGGTCGGGCGC GGGAGGCCTGAAGGGCTCCAATCAGCTGGGCGCGGGCGCGTCGCCG TTCCGGACCCCGCGAGATGGGGACGCCACCAGTTCCCCTCCCCTCGG TTACCGCCCACCCAGCTG |
| | chr20:250627 49-25062900* (VSX1_5_2) | AGGCGCGAGCCTCTCTGGATCCCGTTTGCGGAGGGCCCAGCTTAGAG GAAGCTTTATAGGATTGGGCTGCCGGTCGGGCGCGGAGGCCTGAAG GGCTCCAATCAGCTGGGCGCGGGCGCGTCGCCGTTCCGGACCCCGCG AGATGGGGACG |
| SYT1-Region 1 | chr12:792583 69-79258486 (SYT1_1_1) | GGGCGATGTTGGCTGAACGGATCCACCTATTATTAGTGCGCGGGATT GGCTCCTTGGCGCAGCGTACCCGAGGTGGAGCCAATCAGGATGCTGG GGCTCGGAGATGATGGGCGGCCG |
| | chr12:792583 92-79258498* (SYT1_1_2) | CACCTATTATTAGTGCGCGGGATTGGCTCCTTGGCGCAGCGTACCCG AGGTGGAGCCAATCAGGATGCTGGGGCTCGGAGATGATGGGCGGCCG CCTCCTCACTGG |
| ZNF132-Region 1 | chr19:589512 04-58951524 (ZNF132_1_ 1) | GCTAGCCCTGCCGCTGGGCCGAACCCTCACACTTCCGCTGGGTGACG GTCGCACTCAGGACCTGTCTTCCCAAATGCAAAATGCGCGCAAGGCC TCTGGGCACCGCAGGGACGAAGGCTGGGTATGGAGACCCTGGAGAC GCGTGGCGCTGGCTCCACTTTCGGGAACACCTTGGCTCATAAAAGCA GAGTAATTAGCCGGGCACTATGGTGCGTGTGAAGGCGCGGTGACGGT CCCTGCACCCACTCCCGTGCCCTGGTGTGGCTCCAGAGGCCTGCTGT AGCCCAACCCACCAGCAGCAAAATGAGGACCGCAATGGCG |
| | chr19:589514 02-58951524* (ZNF132_1_ 2) | CGGGCACTATGGTGCGTGTGAAGGCGCGGTGACGGTCCCTGCACCCA CTCCCGTGCCCTGGTGTGGCTCCAGAGGCCTGCTGTAGCCCAACCCA CCAGCAGCAAAATGAGGACCGCAATGGCG |
| ZNF132-Region 2 | chr19:589515 54-58951637 (ZNF132_2_ 1) | CTCGGACTTGCCACTGGTCCGAGCTTTCATTGGCTCAAGGATCCCCGC CGTCTATGACGCTATTTCCCTATGCCCGTCACGCTA |
| ZNF132-Region 3 | chr19:589516 72-58952006 (ZNF132_3_ 1) | CGTTTATTGCGTCGCTGCTCAGGCAACGCAAACTACATTATCCAGAAG GACCCTCGCGGTGCCTCAGGGCTGGCCATTGGCAGCCGAGGAGACAG GCACTTCCGGGCGGAGTGTAAGACGCTGGCCAATCACAGCCTGGCAG CGGGACTTCCGTCGTCGTCCTCGGACCATCACTTTGGCATTTCTCGAT TTTGTCTGCTTCTGAAGGGACCGCGTTGTCAGGCGAGGGACGGAATC TTGGAGGCTCCCTGGGCCCATGGAAACAGGAGCGAGGAAGGCACGA GAGTCGGGGAAGTTCCGCCTTCTTGACATACAAGCGCCCCCACCGCG GCGAA |
| | chr19:589517 24-58951825* (ZNF132_3_ 2) | CTCGCGGTGCCTCAGGGCTGGCCATTGGCAGCCGAGGAGACAGGCAC TTCCGGGCGGAGTGTAAGACGCTGGCCAATCACAGCCTGGCAGCGGG ACTTCCGT |
| VWC2-Region 1 | chr7:4981299 2-49813086 (VWC2 1 1) | AGCCCTGGCAGGGTCTGCGGCCGGCTGCGGAGGTGGGGACGCGGCC GCGCGGACGAGTGACAGGTTGGTCCGGCGCAGCAAAGGAGCGGGT GCG |
| VWC2-Region 2 | chr7:4981305 1-49813343 (VWC2_2_1) | ACAGGTTGGTCCGGCGCAGCAAAGGAGCGGGTGCGGCGGTAGGGC CTCTCCGGGGGCAGCGCGAGGGCCTCCCCCGGGGCAGCGCGGGCCA AGGCGGGGCCTCCAGAGCGGGGGCAGCAGAGGAGGGAGCGGAACG GGAGCCGGGCGGAGGCGGCTGCGGCAGGGGGAGGCGGGAGGCGGG CGCGCTAGCTCCCATGCTGGCCTCGGTGCCACTCGCGCGCCGGCCGC GCTCCGGGCTTCTCTTTCCCTCCGACGCGCCACGGCTGCCCAGACAT TCCGGCTGCCGGGTCT |
| | chr7:4981306 6-49813161* (VWC2_2_1) | GCAGCAAAGGAGCGGGTGCGGCGGTAGGGCCTCTCCGGGGGCAGC GCGAGGGCCTCCCCCGGGGCAGCGCGGGCCAAGGCGGGGCCTCCAG AGCG |
| VWC2-Region 3 | chr7:4981345 5-49813811 (VWC2_3_1) | GGGCGACGCGGGCACTGACGCGAGTTGGGGCCGCGACTACCGGCAG CTGACAGCGCGATGAGCGACTCCCCAGAGACGCCCTAGCCCGGTGTG CGCGCCAGGCGGAGCGCGCAGGTGGGGCTGGGCTGTTAGTGGTCCGC CCCACGCGGGTCGCCGGCCGGCCCAGGATGGGCGCTGGCAACCCGG GCCCGCGCCCGCCGCTGCTACCCCTGCGCCCGCTGCGAGCCCGGCGT CCGGCCCGCGCCCTGCGCTCATGTATGTAGGTTTGGCGCCGGTCTTCA GGGCTGAGGGTGCATGCTGGGTGTCCCCGCGCGGCCGGACGCGCTCC AGCTGGGTTCAGCCGCTCAGCTGCTCCGG |
| | chr7:4981338 7-49813537* (VWC2_3_2) | CCAGGGTGGCCCCGGGCGCGCTTGGTCTCGGAGAAGCGGGGACG AGGCCGGAGGATGAGCGACTGAGGGCGACGCGGGCACTGACGCGAG TTGGGGCCGCGACTACCGGCAGCTGACAGCGCGATGAGCGACTCCCC AGAGACGCCCTA |
| VWC2-Region 4 | chr7:4981472 1-49815073 (VWC2_4_1) | TGTCGTTCTTGGATTTCGCTTCCAATGTATCGCTCCACAGTTTGTGATG TCTAAGAACCCCGGCCGTGCACCGACGCTGGGCATGCTGCCCCCGCC CCCGTCGCCCAGCTCGTTAATCTAGAGCTATGCCGGAGCCCGGGTGGG GGCCGCGGCGGGCCGGGGCGCGCGGGCCGCGGGGCTCAGTTGTG CTGCTGTTCTCTCCGCAGGGACGGCGGCTCCCGGCTGGCGGCGGCGC GCCCCCGGGCTGTGAATGCGACTCGCCCCTCGGCCGCGCTCCCCGCC CGCCCGCCCGCCGGGACGTGGTAGGGGATGCCCAGCTCCACTGCGAT GGCAGTTGGCGCGCTCTCCAGT |
| | chr7:4981476 5-49814936* (VWC2_4_2) | TGATGTCTAAGAACCCCGGCCGTGCACCGACGCTGGGCATGCTGCCC CCGCCCCCGTCGCCCAGCTCGTTAATCTAGAGCTATGCCGGAGCCCGG GTGGGGGCCGCGGCGGGCCGGGGCGCGCGCGGGCCGCGGGGCTCAG TTGTGCTGCTGTTCTCTCCGCAGGGACGGCG |
| VWC2-Region 5 | chr7:4981514 4-49815236 (VWC2_5_1) | AGGCACCAGAGCAGCCGGGCCAGGAGAAGCGTGAGCACGCCTCTCG GGACGGCCCGGGGCGGGTGAACGAGCTCGGGCGCCCGGCGAGGGAC G |
| VWC2-Region 6 | chr7:4981529 0-49815635 (VWC2_6_1) | CGTGAGCCGTGGAGCAAGCTGAAGCAGGCCTGGGTCTCCCAGGGCG GGGGCGCCAAGGCCGGGGATCTGCAGGTCCGGCCCCGCGGGACAC CCCGCAGGCGGAAGCCCTGGCCGCAGCCGCCCAGGACGCGATTGGC CCGGAACTCGCGCCCACGCCCGAGCCACCCGAGGAGTACGTGTACCC GGACTACCGTGGCAAGGGCTGCGTGGACGAGAGCGGCTTCGTGTACG CGATCGGGGAGAAGTTCGCGCCGGGCCCCTCGGCCTGCCCGTGCCTG TGCACCGAGGAGGGGCCGCTGTGCGCGCAGCCCGAGTGCCCGAGGC TGCACCCGCGCTGCATCCACG |
| | chr7:4981534 0-49815439* (VWC2_6_2) | CGCCAAGGCCGGGATCTGCAGGTCCGGCCCGCGGGACACCCCG CAGGCGGAAGCCCTGGCCGCAGCCGCCCAGGACGCGATTGGCCCGG AACTCGCG |

| | | |
|---|---|---|
| Note: * indicates the preferred region for this area. | | |

Table 2 shows the specific primer and probe positions for the amplification regions and the methylation sites they detect, with each primer-probe set covering 7-10 methylation sites. While the probes densely cover methylation sites, the detection scheme designs primers near the 3' end at highly discriminative methylation sites, further enhancing the detection accuracy of the system.

### 2. Design and Selection of Primer and Probe Sequences

Using the bisulfite-treated sequences of the above four gene regions as templates, parameters such as amplicon length, annealing temperature, primer dimer formation, hairpin structures, and specificity were analyzed for different detection regions. This led to the design and selection of primer-probe combinations for each region, with their sequences and identifiers shown in Table 3. All designed primers and probes were synthesized by Ruibo Xingke Biotechnology Co., Ltd., where "F" denotes the forward detection primer, "R" the reverse detection primer, and "P" the detection probe.

**Table 3**

| Gene | Region Range | Primer/Probe Sequence | No . | Sequence (5'-3') |
|---|---|---|---|---|
| VSX1 Region 1 | VSX1_1_1 | VSX1-SEQ1-F | 1 | CGCGTAGGAGTTTAGTAGGTCGTTT |
| | | VSX1-SEQ1-R | 2 | CGAAAAAAAAAATCCGAACCCTAA |
| | | VSX1-SEQ1-P | 3 | CGGCGGAGTTGAGTACGGAGTTTGGTA |
| VSX1 Region 2 | VSX1_2_1 | VSX1-SEQ2-F1 | 4 | CGGGTTTTTTCGATTTACGTAGTT |
| | | VSX1-SEQ2-R1 | 5 | CGATAATCAAACCCGCGCTT |
| | VSX1_2_2 | VSX1-SEQ2-F2 | 6 | ATTATTTTCGTTCGCGTTTTTATTT |
| | | VSX1-SEQ2-R2 | 7 | TCAACGATATAAAACCGCCACC |
| | Same Probe | VSX1-SEQ2-P1/2 | 8 | CGGAGATTGGCGTTTGGCGTTG |
| VSX1 Region 3 | VSX1_3_1 | VSX1-SEQ3-F1 | 9 | TTGGGCGGTAGGAACGGTA |
| | | VSX1-SEQ3-R1 | 10 | AAACTCGCGCCTTACTTACTAAAA |
| | | VSX1-SEQ3-P1 | 11 | TTCGTTAGGAGTAGGTAGGGTGTTCGAGCG |
| | VSX1_3_2 | VSX1-SEQ3-F2 | 12 | TTTTTTTTGTGGTTTCGGTACG |
| | | VSX1-SEQ3-R2 | 13 | CGAAACAACGAACTCGAAACC |
| | | VSX1-SEQ3-P2 | 14 | TCGTCGGCGGTCGTTCGAGTATTTT |
| VSX1 Region 4 | VSX1_4_1 | VSX1_SEQ4_F1 | 15 | AGGAATTATGATCGGTCGGGA |
| | | VSX1_SEQ4_R 1 | 16 | CGAACCCCTAAAAAAACCGC |
| | | VSX1_SEQ4_P1 | 17 | CGTTTTTCGACGGGCGTATTAGTAGTAGGG |
| | VSX1_4_2 | VSX1 SEQ4 F2 | 18 | TTAGCGTTTTGTTGTTAGTGCG |
| | | VSX1_SEQ4_R 2 | 19 | CTAAACCCTCCGCAAACG |
| | | VSX1 SEQ4 P2 | 20 | CGTCGGAAAGCGAGTTTCGGTCGGT |
| VSX1 Region 5 | VSX1_5_1 | VSX1-SEQ5-F1 | 21 | TAGTTGGGTGGGCGGTAATC |
| | | VSX1-SEQ5-R1 | 22 | TCCTTAACAAACAAAACGCGAA |
| | VSX1_5_2 | VSX1-SEO5-F2 | 23 | CGTTTTTATTTCGCGGGGT |
| | | VSX1-SEO5-R2 | 24 | AAACGCGAACCTCTCTAAATCC |
| | Same probe | VSX1-SEQ5-P1/2 | 25 | AGTTTTTTAGGTTTTTCGCGTTCGATCGGT |
| SYT1 Region 1 | SYT1_1_1 | SYT1_SEQ1_F1 | 26 | TATTTATTATTAGTGCGCGGG |
| | | SYT1 SEQ1_R1 | 27 | CCAATAAAAAAACGACCGC |
| | | SYT1_SEQ1_P1 | 28 | CGTAGCGTATTCGAGGTGGAGTTAATTAGGA TGT |
| | SYT1_1_2 | SYT1_SEQ1_F2 | 29 | CGGTCGTTTATTATTTTCGAGTTTTAG |
| | | SYT1_SEQ1_R2 | 30 | AAACGATATTAACTAAACGAATCCACC |
| | | SYT1_SEQ1_P2 | 31 | TGATTGGTTTTATTTCGGGTACGTTGCG |
| ZNF13 2 Region 1 | ZNF132_1_ 1 | ZNF132-SEQ1-F1 | 32 | GTTAGTTTTGTCGTTGGGTCGA |
| | ZNF132_1_ 2 | ZNF_132-SEQ1-F2 | 33 | CGGGTATTATGGTGCGTGTGA |
| | Same reverse primer | ZNF 132-SEQ 1-R1/2 | 34 | CGCCATTACGATCCTCATTTT |
| | Same probe | ZNF 132-SEQ 1-P1/2 | 35 | CGCGGTGACGGTTTTTGTATTTATTTTCG |
| ZNF13 2 Region 2 | ZNF132_2_ 1 | ZNF132_SEQ2_ F | 36 | TAGCGTGACGGGTATAGGGAAA |
| | | ZNR132_SEQ2_ R | 37 | CTCGAACTTACCACTAATCCGAACT |
| | | ZNP132_SEQ2_ P | 38 | AGCGTTATAGACGGCGGGGATTTTTGAG |
| ZNF13 2 Region 3 | ZNF132_3_ 1 | ZNF132-SEQ3-F1 | 39 | CGTTTATTGCGTCGTTGTTTAG |
| | | ZNF132-SEQ3-R1 | 40 | TTCGCCGCGATAAAAACG |
| | ZNF132_3_ 2 | ZNF132-SEQ3-F2 | 41 | TTCGCGGTGTTTTAGGGTT |
| | | ZNF132-SEQ3-R2 | 42 | ACGAAAATCCCGCTACCAAA |
| | Same probe | ZNF132-SEQ3-P1/2 | 43 | CGGGCGGAGTGTAAGACGTTGGTTAATT |
| VWC2 Region 1 | VWC2_1_1 | VWC2_SEQ1-F | 44 | CGTATTCGTTTTTTTTGTTGCG |
| | | VWC2_SEQ1-R | 45 | AACCCTAACAAAATCTACGACCG |
| | | VWC2_SEQ1-P | 46 | CGGATTAATTTGTTATTCGTTCGCGCG |
| VWC2 Region 2 | VWC2_2_1 | VWC2-SEQ2-F1 | 47 | ATAGGTTGGTTCGGCGTAGTAAA |
| | | VWC2-SEQ2-R1 | 48 | AAACCCGACAACCGAAATATCT |
| | VWC2_2_2 | VWC2-SEQ2-F2 | 49 | GTAGTAAAGGGAGCGGGTGC |
| | | VWC2-SEQ2-R2 | 50 | CGCTCTAAAAACCCCGCC |
| | Same probe | VWC2-SEQ2-P1/2 | 51 | CGGGGGTAGCGCGAGGGTTTTTT |
| VWC2 Region 3 | VWC2_3_1 | VWC2-SEQ3-F1 | 52 | TCGGAGTAGTTGAGCGGTTG |
| | | VWC2-SEQ3-R1 | 53 | AAACGACGCGAACACTAACG |
| | VWC2_3_2 | VWC2-SEQ3-F2 | 54 | TAGGGCGTTTTTGGGGAGTC |
| | | VWC2-SEQ3-R2 | 55 | CCAAAATAACCCCCGAAACG |
| | Same probe | VWC2-SEQ3-P1/2 | 56 | CGTTGTTAGTTGTCGGTAGTCGCGGTTTT |
| VWC2 Region 4 | VWC2_4_1 | VWC2-SEQ4-F1 | 57 | TGTCGTTTTTGGATTTCGTTTTT |
| | | VWC2-SEQ4-R1 | 58 | ACTAAAAAACGCGCCAACTACC |
| | VWC2_4_2 | VWC2-SEQ4-F2 | 59 | TGATGTTTAAGAATTTCGGTCGT |
| | | VWC2-SEQ4-R2 | 60 | CGCCGTCCCTACGAAAAAA |
| | Same probe | VWC2-SEQ4-P1/2 | 61 | CGACGTTGGGTATGTTGTTTTCGTTTTCG |
| VWC2 Region 5 | VWC2_5_1 | VWC2_SEQ5_F | 62 | CGTTTTTCGTCGGGCG |
| | | VWC2_SEQ5_R | 63 | AAACACCAAAACAACCGAACC |
| | | VWC2_SEQ5_P | 64 | TCGAGTTCGTTTATTCGTTTCGGGTCG |
| VWC2 Region 6 | VWC2_6_1 | VWC2-SEQ6-F1 | 65 | CGTGAGTCGTGGAGTAAGTTGAA |
| | | VWC2-SEQ6-R1 | 66 | CGTAAATACAACGCGAATACAACCT |
| | VWC2_6_2 | VWC2-SEQ6-F2 | 67 | CGTTAAGGTCGGGGATTTGTA |
| | | VWC2-SEQ6-R2 | 68 | CGCGAATTCCGAACCAAT |
| | Same probe | VWC2-SEQ6-P1/2 | 69 | TTCGGTTTCGCGGGGATATTTCGTA |

Among these, both ends of the probe sequences are labeled with modification groups, including a 5' group and a 3' group. The 5' group is selected from any one of FAM, VIC, HEX, NED, ROX, TET, JOE, TAMRA, CY3, and CY5; in this embodiment, CY5 is used for the target gene and FAM for the reference gene. The 3' group is selected from any one of MGB, BHQ-1, BHQ-2, BHQ-3, IBRQ, and MGB-NFQ; in this embodiment, BHQ-2 was used for the target gene and BHQ-1 for the reference gene.

3. The kit components for sample testing are as follows: PCR reaction solution: 2×Luna^{®} Universal Probe qPCR Master Mix (New England Biolabs (NEB), catalog number: M3004E); primer-probe mixture; positive control: synthesized methylated plasmid fragment of the target gene (Tsingke); negative control: synthesized unmethylated plasmid fragment of the target gene (Tsingke); blank control: nuclease-free water.

4. Sample testing: the methylation markers discovered in this disclosure are specific markers for endometrial cancer and can be used for testing various biological samples, including but not limited to tissues, urine, swabs, brushings, blood, etc. This disclosure will further elaborate on its application scenarios. The present disclosure detected DNA methylation in samples; prior to detecting methylation signals, DNA extraction from various samples and bisulfite conversion of the extracted DNA were required for subsequent quantitative fluorescent PCR detection.

### 4.1 Sample collection

4.1.1 Tissue sample collection: after obtaining informed consent from patients undergoing total hysterectomy and bilateral salpingo-oophorectomy, postoperative gross cancerous tissue approximately the size of a mung bean was taken as the cancer tissue sample, and tissue within 3cm from the edge of the cancer lesion was taken as the adjacent non-cancerous tissue sample. After collection, samples were placed in cryotubes and stored in a -80°C freezer.

4.1.2 Urine sample collection: subjects were instructed to use a 50mL urine cup to collect the first portion of the first morning urine, then pour the urine into a urine collection tube pre-filled with Urine Conditioning Buffer (zymo, catalog number: D3061-1-140), collecting 40-50mL of urine for testing.

4.1.3 Cervical brushing sample collection: uniformly trained medical personnel, assisted by a speculum, inserted a cervical brush into the cervix, rotated it clockwise five times to collect cervical cells, and placed the brush into an exfoliated cell collection bottle containing 2mL PBS, followed by low-temperature storage and transport.

4.1.4 Vaginal swab sample collection: uniformly trained medical personnel used a sterile swab to rotate and sample at the upper 1/3 of the vaginal sidewall, ensuring secretions were visibly attached to the swab. The swab with secretions was then placed into an exfoliated cell collection tube pre-filled with 2mL PBS, followed by low-temperature storage and transport.

### 4.2 Sample DNA Extraction

4.2.1 Tissue/brush/swab samples: a Blood/Cell/Tissue Genomic DNA Extraction Kit (TIANGEN, Cat. No.: DP304-03) was used as the reagent for tissue/brush/swab genomic DNA extraction.

4.2.2 Urine samples: a Quick-DNA^{™} Urine Kit (zymo, Cat. No.: D3061) was used to extract DNA from urine samples.

### 4.3 Sample DNA Bisulfite Conversion

The DNA bisulfite conversion kit was purchased from Zymo. Following the kit instructions, extracted DNA was subjected to bisulfite conversion, where unmethylated cytosines (C) were converted to uracil (U), while methylated cytosines (C) remain unchanged, yielding converted bis-DNA.

### 4.4 Fluorescence Quantitative PCR Amplification of Bis-DNA

Different primer-probe combinations from Table 2 were used to amplify and detect corresponding distinct regions, as detailed below:

4.4.1 Preparation of PCR reaction liquid and primer-probe mixture: 5µL of 2×EpiTect MethyLight Master Mix; forward detection primer F 0.3-0.5µmol; reverse detection primer R 0.3-0.5µmol; detection probe P 0.1-0.3µmol; sample DNA <100ng; add purified water to a total volume of 10µL.

4.4.2 Sample loading: the prepared mixture was dispensed into a 96-well/384-well plate, 8µL per well, then Bis-DNA was add to the sample wells, with a loading volume of 2µL per sample well, using one replicate per sample. Three control samples were included: positive control, negative control, and no-template control (NTC), loaded using the same method.

4.4.3 Fluorescence quantitative PCR amplification detection: sample detection was carried out using the ROCHE 480 fluorescence quantitative PCR instrument. The sample amplification program was set as follows: first 95°C 60s; then 95°C 15s, 55-65°C* 30s (fluorescence signal collection), for 40-50 cycles*; finally 4°C 30s (*adjust appropriately based on specific gene and sample type).

4.4.4 Signal collection: FAM and CY5 signals wer collected at 55-65°C.

### 5. Interpretation of Detection Results

5.1 Assay System Evaluation: the test result was considered valid if the positive control shows a normal amplification curve, and the negative control and no-template control samples showed no amplification curve.

### 5.2 Sample Test Result Evaluation

5.2.1 The result was valid if the internal control channel showed a S-type amplification curve and the Ct value was ≤30;

### 5.2.2 A Ct value of 50 was assigned for no amplification.

5.2.3 The methylation levels of the 4 genes were represented by the M-index = 2^(-ΔCt) value: $M - index \left(VSX1\right) = {2}^{∧} \left(Ct\left(ACTB\right)-Ct\left(VSX1\right)\right) ;$ $M - index \left(SYT1\right) = {2}^{∧} \left(Ct\left(ACTB\right)-Ct\left(SYT1\right)\right) ;$ $M - index \left(ZNF132\right) = {2}^{∧} \left(Ct\left(ACTB\right)-Ct\left(ZNF132\right)\right) ;$ $M - index \left(VWC2\right) = {2}^{∧} \left(Ct\left(ACTB\right)-Ct\left(VWC2\right)\right) .$

### 6. Assay System Evaluation

To evaluate the stability of each test reaction plate, this kit constructed a standard with 10²-10⁷ copies/reaction well using artificially synthesized sequences corresponding to fully methylated amplification products. By detecting the Ct values of serial standards in each reaction plate during every test, a standard curve could be constructed. The stability of the test could be evaluated by comparing the standard curves across different test plates.

### 7. Detection Performance Evaluation

After PCR testing, the Ct values for the methylation sites of the ACTB, VSX1, VWC2, ZNF132, and SYT1 genes could be obtained for each sample, with a Ct value of 50 assigned for no amplification. The M-index for each sample's target gene was calculated using the above formulas. For different sample types (tissue/urine/brush/smear), a case-control Logistic model was sequentially constructed using the M-index of the four methylation sites detected. The endometrial cancer risk score and the threshold for determining endometrial cancer occurrence were calculated for each sample from the fitted equation. Samples were classified into endometrial cancer positive and negative groups by comparing their scores with the threshold. The grouping based on the M-index Logistic model was compared with the sample clinicopathological grouping to obtain an ROC curve for evaluating the diagnostic performance of the methylation degree model. The area under the curve (AUC), sensitivity, and specificity were calculated from the ROC curve. The detection efficacy of the reagent of the present disclosure could be judged based on the AUC/sensitivity/specificity metrics.

### Example 3: Detection of Tissue Samples by Primer-Probe Combination

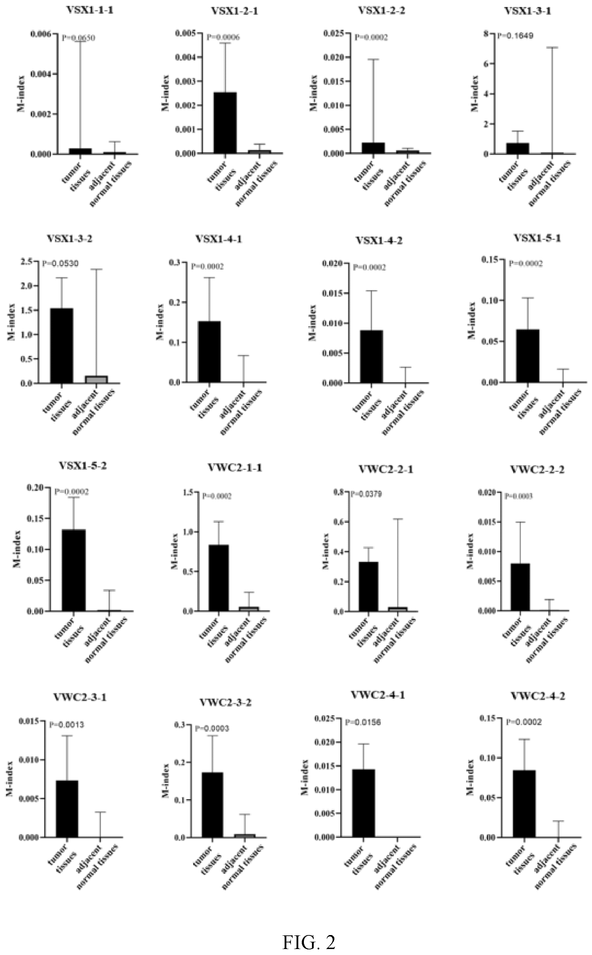

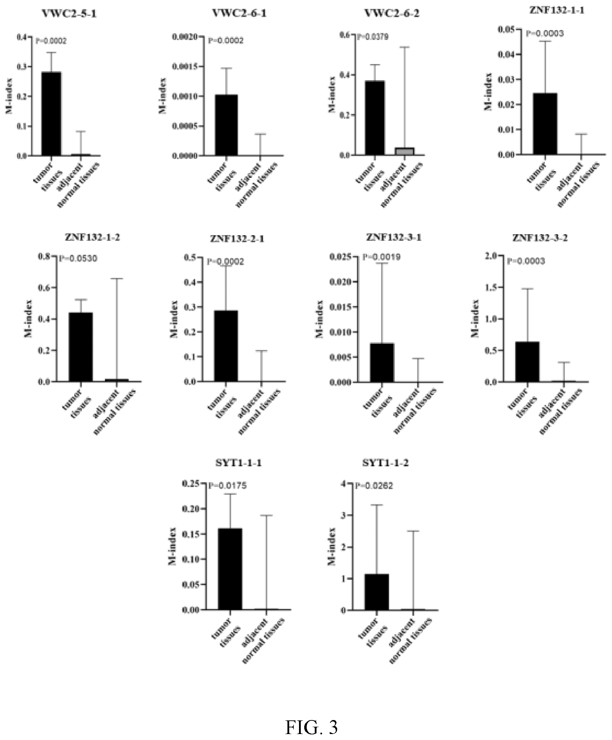

To further optimize the primer-probe combinations in Example 2, clinicians collected 8 pairs of cancerous lesion tissues and adjacent normal tissues from postoperative gross specimens of endometrial cancer patients for testing. After extraction, conversion, and detection of the tissue specimens using the method described in Example 2, the M-index (i.e. 2^(-ΔCt)) of different genes for each sample was obtained, with results shown in FIGS. 2-3. As seen in the figures, compared to adjacent normal tissues, the methylation levels at the detection sites of VSX1, SYT1, ZNF132, and VWC2 genes in cancerous tissue samples from endometrial cancer patients were significantly higher, showing a notable difference.

### Example 4: Detection of Shed Cell Samples Using Primer-Probe Combinations

In this example, 32 shed cell samples from endometrial cancer patients and 33 non-cancerous controls were collected and tested. Results showed that the AUC for each primer-probe combination in distinguishing endometrial cancer from the control group was greater than 0.7, demonstrating good diagnostic performance, as detailed in Table 4. Among them, detection regions such as VSX1_4_2, VSX1_5_2, VWC2_2_2, VWC2_4_2, VWC2_5_1, VWC2_6_2, ZNF132_1_1, ZNF132_2_1, ZNF132_3_2, and SYT1_1_2 achieved AUCs above 0.8, indicating that multiple regions of these genes hold diagnostic value as biomarkers for endometrial cancer.

**Table 4**

| Detection Area | AUC | Sensitivity (%) | Specificity (%) | Youden Index |
|---|---|---|---|---|
| VSX1 1 1 | 0.615 | 46.9 | 81.8 | 0.287 |
| VSX1 2 1 | 0.798 | 71.9 | 84.8 | 0.567 |
| VSX1 2 2 | 0.770 | 59.4 | 84.8 | 0.442 |
| VSX1 3 1 | 0.667 | 50.0 | 78.8 | 0.288 |
| VSX1 3 2 | 0.700 | 59.4 | 84.8 | 0.442 |
| VSX1 4 1 | 0.781 | 70.6 | 85.7 | 0.563 |
| VSX1 4 2 | 0.844 | 84.4 | 72.7 | 0.571 |
| VSX1 5 1 | 0.742 | 56.3 | 91.0 | 0.472 |
| VSX1 5 2 | 0.841 | 75.0 | 87.9 | 0.629 |
| VWC2 1 1 | 0.855 | 75.7 | 83.9 | 0.595 |
| VWC2 2 1 | 0.682 | 68.8 | 72.7 | 0.415 |
| VWC2 2 2 | 0.828 | 62.5 | 97.0 | 0.595 |
| VWC2 3 1 | 0.627 | 34.4 | 90.9 | 0.253 |
| VWC2 3 2 | 0.710 | 50.0 | 90.9 | 0.409 |
| VWC2 4 1 | 0.610 | 46.9 | 75.8 | 0.226 |
| VWC2 4 2 | 0.858 | 81.3 | 81.8 | 0.631 |
| VWC2 5 1 | 0.864 | 78.1 | 97.0 | 0.751 |
| VWC2 6 1 | 0.654 | 40.6 | 87.9 | 0.285 |
| VWC2 6 2 | 0.903 | 87.5 | 87.9 | 0.754 |
| ZNF132 1 1 | 0.804 | 75.0 | 81.8 | 0.568 |
| ZNF132 1 2 | 0.689 | 46.9 | 87.9 | 0.348 |
| ZNF132 2 1 | 0.895 | 75.0 | 93.9 | 0.689 |
| ZNF132 3 1 | 0.680 | 56.3 | 75.8 | 0.320 |
| ZNF132 3 2 | 0.846 | 71.9 | 93.9 | 0.658 |
| SYT1 1 1 | 0.775 | 75.0 | 84.8 | 0.598 |
| SYT1 1 2 | 0.801 | 81.3 | 72.7 | 0.540 |

Considering evaluation metrics such as AUC, sensitivity, and specificity, VSX1_4_2 (Chr20: 25062655-25062739), VWC2_5_1 (Chr7:49815144-49815236), ZNF132_2_1 (Chr19:58951554-58951637), and SYT1_1_2 (Chr12: 79258392-79258498) were selected as the representative regions for these four genes, respectively, for testing in urine samples.

### Example 5: Detection of Urine Samples

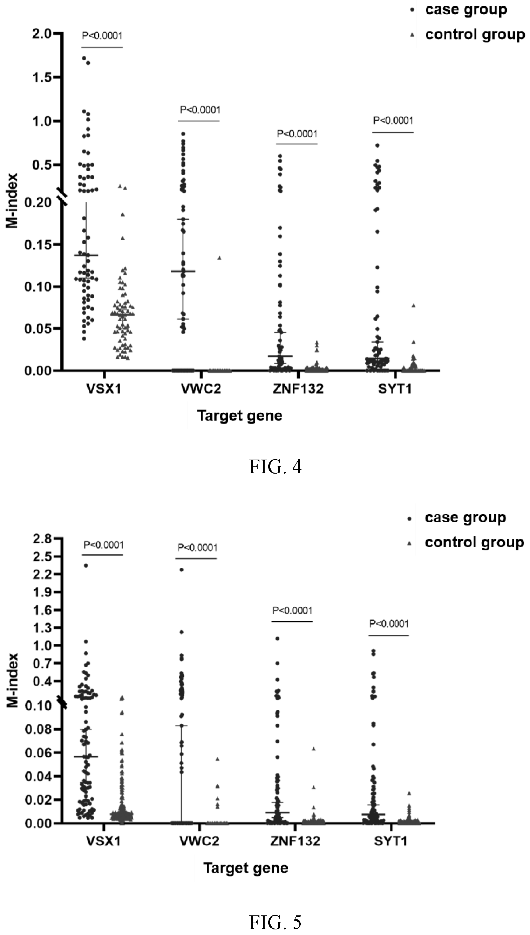

A total of 133 urine samples were collected at the Sun Yat-sen University Cancer Center, including 65 preoperative urine samples from patients histopathologically diagnosed with endometrial cancer, 16 preoperative urine samples from patients with benign gynecological diseases (including uterine fibroids and ovarian chocolate cysts), and 52 urine samples from healthy volunteers (with no abnormalities detected in physical examinations within the past year). Based on whether the subjects had endometrial cancer, the urine samples were divided into a case group (65 endometrial cancer patients) and a control group (68 patients with benign gynecological diseases and healthy volunteers). After extraction, conversion, and detection of the urine samples using the method described in Example 2, the M-index for different genes in each sample was obtained. The detection results are shown in the scatter plot in FIG. 4, indicating that the gene loci discovered in this disclosure can also effectively distinguish between the case group and the control group in urine samples, with the methylation levels in the case group being significantly higher than those in the control group. Furthermore, modeling analysis was conducted according to the detection performance evaluation method described in Example 2, with the analysis results as follows.

Through further modeling analysis, the detection performance of the single-gene methylation site model is shown in the table below.

**Table 5**

| Single-gene indicator | AUC | Sensitivity (%) | Specificity (%) | Youden index |
|---|---|---|---|---|
| VSX1(chr20:25062655-25062739) | 0.858 | 81.5 | 77.9 | 0.594 |
| VWC2(chr7:49815144-49815236) | 0.827 | 70.8 | 98.5 | 0.693 |
| ZNF132(chr19:58951554-58951637) | 0.808 | 67.7 | 86.8 | 0.545 |
| SYT1(chr12:79258392-79258498) | 0.840 | 76.9 | 82.4 | 0.593 |

As shown in the table, the detection method constructed in this disclosure demonstrates excellent discriminative ability between the case group and control group in urine samples, with AUCs for single-gene indicators all above 0.8 after modeling. Among the single-gene indicators, the VSX1 gene exhibits the strongest overall discriminative power, with an AUC of 0.858. At a sensitivity of 81.5%, the corresponding specificity reaches 77.9%. Different genes show distinct advantages in discrimination: the methylation site of the VSX1 gene has high sensitivity, with a sensitivity as high as 81.5% at its optimal cutoff, indicating it can identify more cancer cases; whereas the methylation site of the VWC2 gene has high specificity, with a sensitivity as high as 98.5% at its optimal cutoff, enabling the identification of more non-cancer patients. Furthermore, combining two/three/four methylation indicators of the four genes for joint detection analysis yields the results shown in Table 6.

| Joint indicator modeling | | AUC | Sensitivity (%) | Specificity (%) | Youden index |
|---|---|---|---|---|---|
| Two-indicator model | VSX1+ZNF132 | 0.875 | 75.4 | 83.8 | 0.592 |
| | VSX1+VWC2 | 0.928 | 84.6 | 88.2 | 0.728 |
| | VSX1+SYT1 | 0.878 | 72.3 | 91.2 | 0.635 |
| | VWC2+ZNF132 | 0.849 | 70.8 | 98.5 | 0.693 |
| | ZNF132+SYT1 | 0.854 | 78.5 | 80.9 | 0.594 |
| | VWC2+SYT1 | 0.907 | 89.2 | 83.8 | 0.730 |
| Three-indicator model | VSX1+ZNF132+VWC2 | 0.927 | 84.6 | 88.2 | 0.728 |
| | VSX1+ZNF132+SYT1 | 0.880 | 78.5 | 83.8 | 0.623 |
| | VSX1+VWC2+SYT1 | 0.932 | 86.2 | 89.7 | 0.759 |
| | ZNF132+VWC2+SYT1 | 0.902 | 86.2 | 83.8 | 0.700 |
| Four-indicator model | VSX1+ZNF132+VWC2+SYT1 | 0.930 | 84.6 | 91.2 | 0.758 |

As shown in the table above, among the 133 urine samples collected at Sun Yat-sen University Cancer Center, the model constructed by combining 2-4 gene methylation markers demonstrated further improved discrimination between endometrial cancer cases and controls. The AUC of the multi-marker combined model could reach as high as 0.932 (combining VSX1+VWC2+SYT1 markers). With only two markers combined, the AUC for the VSX1 and VWC2 combination reached 0.928, with a sensitivity of 84.6% and specificity of 88.2%. The model combining VWC2 and SYT1 markers achieved a sensitivity of 89.2%; the model combining VWC2 and ZNF132 markers achieved a specificity as high as 98.5%. These results indicate a complementary effect among the markers, and combining multiple markers can complement their advantages, with the performance of multi-marker combined models surpassing that of single-marker models.

### Example 6: Collection of Multicenter Urine Samples for Testing

To evaluate the performance stability of the method constructed in the present disclosure for urine sample testing, further, 336 urine samples from endometrial cancer patients and non-cancer individuals were collected from other centers: Guangzhou Medical University Affiliated Cancer Hospital and Foshan First People's Hospital, including 93 samples from endometrial cancer patients (case group) and 243 samples from non-cancer patients (control group). The specific testing method was the same as in Example 2. After testing, the M-index of different genes for each sample was obtained. The test results are shown in the scatter plot in FIG. 5. The gene methylation markers discovered in the present disclosure also achieved the effect of distinguishing the case group from the control group in multicenter-collected urine samples, with the methylation level of the case group significantly higher than that of the control group. Further, modeling analysis was performed according to the detection performance evaluation method described in Example 2, and the analysis results are as follows.

As shown in Table 7, the detection method constructed in the present disclosure still has good discrimination ability between the case group and control group in multicenter-collected urine samples. The AUC after single-gene marker modeling ranged from 0.779 to 0.879, with different genes showing different discrimination advantages. In multicenter-collected urine samples, the ZNF132 marker had good discrimination ability, with an AUC of 0.879, and a sensitivity of 64.5% at a specificity of 94.7%. The methylation marker of the VSX1 gene still had high sensitivity, with a sensitivity as high as 81.7% at a specificity of 78.6%, indicating its ability to identify more cancer cases. In summary, the single methylation markers discovered in the present disclosure still have good detection performance in multicenter-collected urine samples. Further, two/three/four-marker combined analysis of the methylation markers of the four genes yielded the results shown in Table 8 below.

**Table 7**

| Single-gene indicator | AUC | Sensitivity (%) | Specificity (%) | Youden index |
|---|---|---|---|---|
| VSX1(chr20:25062655-25062739) | 0.862 | 81.7 | 78.6 | 0.603 |
| VWC2(chr7:49815144-49815236) | 0.779 | 48.4 | 99.6 | 0.480 |
| ZNF132(chr19:58951554-58951637) | 0.879 | 64.5 | 94.7 | 0.592 |
| SYT1(chr12:79258392-79258498) | 0.863 | 78.5 | 83.1 | 0.616 |

**Table 8**

| Joint indicator modeling | | AUC | Sensitivity (%) | Specificity (%) | Youden index |
|---|---|---|---|---|---|
| Two-indicator model | VSX1+ZNF132 | 0.892 | 69.9 | 90.5 | 0.604 |
| | VSX1+VWC2 | 0.873 | 82.8 | 77.0 | 0.598 |
| | VSX1+SYT1 | 0.863 | 81.7 | 78.6 | 0.603 |
| | VWC2+ZNF132 | 0.885 | 86.0 | 73.7 | 0.597 |
| | ZNF132+SYT1 | 0.845 | 67.7 | 91.8 | 0.595 |
| | VWC2+SYT1 | 0.618 | 48.4 | 99.6 | 0.480 |
| Three-marker model | VSX1+ZNF132+VWC2 | 0.885 | 71.0 | 89.3 | 0.603 |
| | VSX1+ZNF132+SYT1 | 0.893 | 80.6 | 79.8 | 0.604 |
| | VSX1+VWC2+SYT1 | 0.874 | 82.8 | 77.0 | 0.598 |
| | ZNF132+VWC2+SYT1 | 0.854 | 86.0 | 74.1 | 0.601 |
| Four-marker model | VSX1+ZNF132+VWC2+SYT1 | 0.886 | 71.0 | 89.3 | 0.603 |

As shown in the table above, among the 336 urine samples collected from multiple centers, the model constructed by combining 2-4 gene methylation markers improved the discrimination between endometrial cancer cases and controls. After multi-marker combination, the AUC of the models, except for the VWC2+SYT1 combination, all exceeded 0.8, with the highest AUC reaching 0.893 for the three-marker combined model of VSX1+ZNF132+SYT1. This model achieved a specificity of 79.8% at a sensitivity of 80.6%. The above results indicate that among urine samples collected from multiple centers, these markers have a certain complementary effect, and combining multiple markers can further enhance the detection efficacy for endometrial cancer.

### Example 7: Differentiation between Endometrial Cancer and Benign Diseases

In actual clinical practice, patients with endometrial cancer often present with symptoms of abnormal vaginal bleeding in the early stages, but this symptom is not specific, as most patients with benign gynecological diseases in women of reproductive age and postmenopausal women also experience abnormal vaginal bleeding. Therefore, the present disclosure further evaluates the ability of this detection method to differentiate between patients with endometrial cancer and those with benign gynecological diseases. By defining urine samples from 158 patients with endometrial cancer across multiple centers as the case group and urine samples from 236 patients with benign gynecological diseases as the control group, the specific conditions of benign gynecological diseases were as follows: 13 cases of endometrial hyperplasia; 60 cases of gynecological inflammation (including cervicitis, endometritis); 103 cases of uterine polyps; 32 cases of uterine fibroids; 28 cases of other conditions (including infertility, pelvic inflammatory disease, etc.).

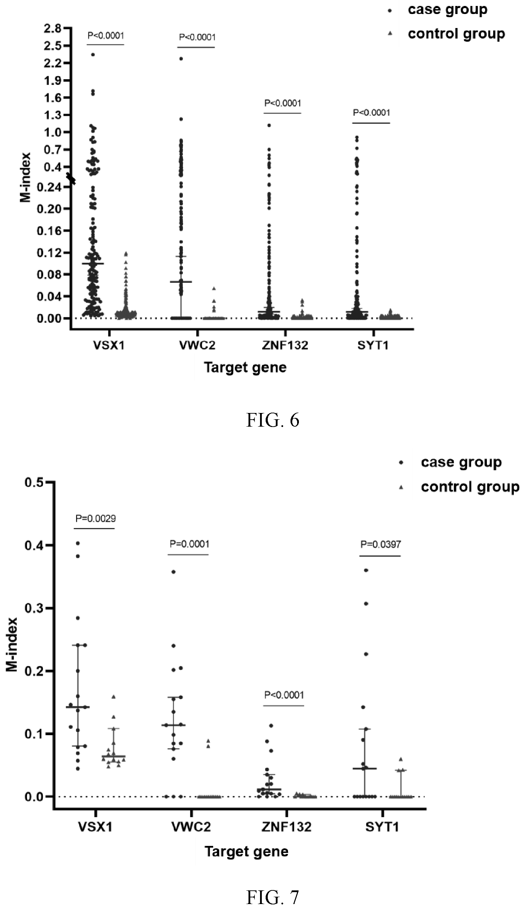

The specific detection method was the same as in Example 2. After testing, the M-index for different genes in each sample was obtained. The test results are shown in the scatter plot in FIG. 6. The gene methylation markers discovered in the present disclosure showed significant differences between patients with endometrial cancer and those with benign gynecological diseases, with methylation levels in the case group being markedly higher than those in the control group. Further, modeling analysis was conducted according to the detection efficacy evaluation method described in Example 2, and the analysis results are as follows.

As shown in Table 9, the detection method constructed in the present disclosure demonstrates excellent discriminative ability between endometrial cancer patients and benign disease patients in multicenter-collected urine samples. The AUC of the single-gene indicator model ranges from 0.831 to 0.916. The VSX1 indicator shows good capability in distinguishing endometrial cancer patients from patients with benign gynecological diseases, with an AUC of 0.916, achieving a sensitivity of 88.0% at a specificity of 82.2%. The methylation indicator of the VWC2 gene still maintains high specificity, reaching a sensitivity of 63.9% at a specificity of 97.5%, with an AUC of 0.831. In summary, the single methylation markers discovered in the present disclosure can effectively distinguish endometrial cancer patients from other benign gynecological disease patients in multicenter-collected urine samples. Furthermore, combining the methylation indicators of the four genes for two-/three-/four-indicator joint analysis yields the results shown in Table 10 below.

**Table 9**

| Single-gene indicator | AUC | Sensitivity (%) | Specificity (%) | Youden index |
|---|---|---|---|---|
| VSX1(chr20:25062655-25062739) | 0.916 | 88.0 | 82.2 | 0.702 |
| VWC2(chr7:49815144-49815236) | 0.831 | 63.9 | 97.5 | 0.614 |
| ZNF132(chr19:58951554-58951637) | 0.885 | 73.4 | 90.7 | 0.641 |
| SYT1(chr12:79258392-79258498) | 0.880 | 69.0 | 94.1 | 0.631 |

**Table 10**

| Joint indicator modeling | | AUC | Sensitivity (%) | Specificity (%) | Youden index |
|---|---|---|---|---|---|
| Two-indicator model | VSX1+ZNF132 | 0.927 | 81.0 | 90.7 | 0.717 |
| | VSX1+VWC2 | 0.925 | 83.5 | 86.0 | 0.695 |
| | VSX1+SYT1 | 0.917 | 88.0 | 82.2 | 0.702 |
| | VWC2+ZNF132 | 0.897 | 75.9 | 88.6 | 0.645 |
| | ZNF132+SYT1 | 0.853 | 73.4 | 91.1 | 0.645 |
| | VWC2+SYT1 | 0.692 | 60.8 | 97.5 | 0.583 |
| Three-indicator model | VSX1+ZNF132+VWC2 | 0.926 | 82.3 | 88.1 | 0.704 |
| | VSX1+ZNF132+SYT1 | 0.927 | 81.0 | 90.7 | 0.717 |
| | VSX1+VWC2+SYT1 | 0.925 | 83.5 | 86.0 | 0.695 |
| | ZNF132+VWC2+SYT1 | 0.684 | 36.7 | 100.0 | 0.367 |
| Four-indicator model | VSX1+ZNF132+VWC2+SYT1 | 0.835 | 84.8 | 82.2 | 0.670 |

As shown in the table above, among the 394 urine samples collected from multiple centers, the model constructed by combining 2-4 gene methylation markers improved the discrimination between endometrial cancer cases and benign gynecological diseases. The AUC of the multi-marker combined model reached up to 0.927, with both the two-marker combined model (VSX1+ZNF132) and the three-marker combined model (VSX1+ZNF132+SYT1) achieving an AUC of 0.927, corresponding to a sensitivity of 81.0% at a specificity of 90.7%. These results indicate that among urine samples collected from multiple centers, the above markers have complementary effects, and multi-marker combination can further enhance the detection efficacy in distinguishing endometrial cancer from benign gynecological diseases.

In summary, through evaluation using urine samples collected at this center and further expanded testing with multi-center urine samples, the superior detection performance of the gene methylation markers protected by this disclosure has been demonstrated. Each marker has its own advantages, and multi-marker combination achieves complementary strengths, further improving detection performance. Additionally, the present disclosure's ability to discriminate between endometrial cancer and benign gynecological diseases in urine samples was further analyzed, showing good performance advantages in differential diagnosis. All of the above proves that the gene methylation markers discovered in this disclosure can detect endometrial cancer with high accuracy using urine samples.

### Example 8: Detection of Cervical Brush Samples

Furthermore, to evaluate the detection efficacy of the gene methylation markers discovered in this disclosure in cervical brush samples, 17 cervical brush samples from patients with histopathologically confirmed endometrial cancer and 14 from healthy volunteers were collected from Foshan First People's Hospital, with sample collection as described in Example 2. The 17 samples from endometrial cancer patients were defined as the case group, and the 14 samples from healthy volunteers as the control group. The specific detection method was as described in Example 2, and the M-index for different genes in each sample was obtained after testing. The results are shown in the scatter plot in FIG. 7. In the brush samples, the gene methylation indicators discovered in this disclosure showed significant differences between endometrial cancer patients and healthy volunteers, with methylation levels in the case group significantly higher than those in the control group. Further, modeling analysis was conducted according to the detection performance evaluation method described in Example 2, with the analysis results as follows. As shown in the table, the detection method constructed in this disclosure demonstrated excellent discriminative ability between endometrial cancer patients and healthy volunteers in brush samples. The AUC for single-gene indicator models ranged from 0.718 to 0.891. The ZNF132 indicator showed good discriminative ability, with an AUC of 0.891, and a sensitivity of 76.5% at a specificity of 92.9%. The detection performance of the VWC2 gene was second only to that of ZNF132, with an AUC of 0.882 and a sensitivity of 88.2% at a specificity of 85.7%. SYT1 exhibited high specificity at 92.9%.

**Table 11**

| Single-gene indicator | AUC | Sensitivity (%) | Specificity (%) | Youden index |
|---|---|---|---|---|
| VSX1(chr20:25062655-25062739) | 0.807 | 82.4 | 71.4 | 0.538 |
| VWC2(chr7:49815144-49815236) | 0.882 | 88.2 | 85.7 | 0.739 |
| ZNF132(chr19:58951554-58951637) | 0.891 | 76.5 | 92.9 | 0.694 |
| SYT1(chr12:79258392-79258498) | 0.718 | 52.9 | 92.9 | 0.458 |

Furthermore, a joint analysis of two/three/four methylation markers from the four genes was conducted, yielding the results shown in the table below.

**Table 12**

| Joint indicator modeling | | AUC | Sensitivity (%) | Specificity (%) | Youden index |
|---|---|---|---|---|---|
| Two-indicator model | VSX1+VWC2 | 0.861 | 82.4 | 85.7 | 0.681 |
| | VSX1+ZNF132 | 0.845 | 82.4 | 92.9 | 0.753 |
| | VSX1+SYT1 | 0.836 | 88.2 | 71.4 | 0.596 |
| | VWC2+ZNF132 | 0.912 | 82.4 | 92.9 | 0.753 |
| | VWC2+SYT1 | 0.861 | 82.4 | 85.7 | 0.681 |
| | ZNF132+SYT1 | 0.895 | 82.4 | 100.0 | 0.824 |
| Three-indicator model | VSX1+VWC2+ZNF132 | 0.899 | 76.5 | 100.0 | 0.765 |
| | VSX1+VWC2+SYT1 | 0.849 | 82.4 | 85.7 | 0.681 |
| | VSX1+ZNF132+SYT1 | 0.857 | 76.5 | 100.0 | 0.765 |
| | VWC2+ZNF132+SYT1 | 0.916 | 76.5 | 100.0 | 0.765 |
| Four-indicator model | VSX1+VWC2+ZNF132+SYT1 | 0.912 | 76.5 | 100.0 | 0.765 |

As shown in the table above, among the 31 brushing samples, the model constructed by combining 2-4 gene methylation indicators further improved the discrimination ability between endometrial cancer cases and controls. After multi-indicator combination, the model's AUC could reach as high as 0.916 (combining VWC2+ZNF132+SYT1 indicators). At a model specificity of 100%, the corresponding sensitivity was 76.5%. The above results indicate that the indicators also have complementary effects in brush samples. Combining multiple indicators can leverage their respective strengths, and the performance of the multi-indicator combined model is superior to that of single-indicator models.

### Example 9: Detection of vaginal swab samples

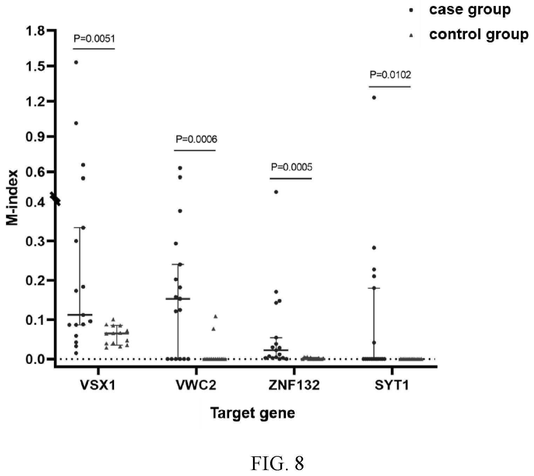

As described in Example 8, vaginal swab samples were collected from the same population as the cervical brushing samples in Example 8, with sample collection and processing as described in Example 2. By defining swab samples from 17 endometrial cancer patients as the case group and swab samples from 14 healthy volunteers as the control group. The specific detection method was as described in Example 2, and after detection, the M-index of different genes for each sample was obtained. The detection results are shown in the scatter plot in FIG. 8. In the swab samples, the gene methylation indicators discovered in the present disclosure showed significant differences between endometrial cancer patients and healthy volunteers, with the methylation level in the case group being significantly higher than that in the control group. Furthermore, modeling analysis was conducted according to the detection effect evaluation method described in Example 2, with the analysis results as follows. As shown in Table 13, the detection method constructed in the present disclosure demonstrated good discrimination ability between endometrial cancer patients and healthy volunteers in swab samples. The AUC of single-gene indicator models ranged from 0.769 to 0.853, with the ZNF132 indicator showing better discrimination ability, having an AUC of 0.853 and a sensitivity of 64.7% at 100% specificity. The detection effect of the VWC2 gene was second only to that of the ZNF132 gene, with an AUC of 0.849 and a sensitivity of 64.7% at 100% specificity. Furthermore, by conducting two/three/four-indicator combined analysis of the methylation indicators of the four genes, the results shown in Table 14 below were obtained.

**Table 13**

| Single-gene metric | AUC | Sensitivity (%) | Specificity (%) | Youden index |
|---|---|---|---|---|
| VSX1(chr20:25062655-25062739) | 0.790 | 76.5 | 85.7 | 0.622 |
| VWC2(chr7:49815144-49815236) | 0.849 | 64.7 | 100.0 | 0.647 |
| ZNF132(chr19:58951554-58951637) | 0.853 | 64.7 | 100.0 | 0.647 |
| SYT1(chr12:79258392-79258498) | 0.769 | 76.5 | 64.3 | 0.408 |

**Table 14**

| Joint indicator modeling | | AUC | Sensitivity (%) | Specificity (%) | Youden index |
|---|---|---|---|---|---|
| Two-indicator model | VSX1+VWC2 | 0.798 | 64.7 | 100.0 | 0.647 |
| | VSX1+ZNF132 | 0.857 | 64.7 | 100.0 | 0.647 |
| | VSX1+SYT1 | 0.832 | 82.4 | 85.7 | 0.681 |
| | VWC2+ZNF132 | 0.903 | 70.6 | 100.0 | 0.706 |
| | VWC2+SYT1 | 0.874 | 70.6 | 100.0 | 0.706 |
| | ZNF132+SYT1 | 0.962 | 88.2 | 100.0 | 0.882 |
| Three-indicator model | VSX1+VWC2+ZNF132 | 0.908 | 82.4 | 92.9 | 0.753 |
| | VSX1+VWC2+SYT1 | 0.836 | 70.6 | 100.0 | 0.706 |
| | VSX1+ZNF132+SYT1 | 0.966 | 82.4 | 100.0 | 0.824 |
| | VWC2+ZNF132+SYT1 | 0.962 | 88.2 | 100.0 | 0.882 |
| Four-indicator model | VSX1+VWC2+ZNF132+SYT1 | 0.966 | 82.4 | 100.0 | 0.824 |

As shown in the table above, among the 31 swab samples, the model constructed by combining 2-4 gene methylation markers significantly improved the discrimination between endometrial cancer cases and controls. After multi-marker combination, the model's AUC could reach as high as 0.966 (combining VSX1+ZNF132+SYT1 markers or the four-marker combination), with a sensitivity of 88.2% when specificity was 100%. These results indicate that the markers in swab samples also have a complementary effect; combining multiple markers can complement their respective strengths, and the performance of the multi-marker combined model is superior to that of single-marker models.

In summary, the gene methylation markers discovered in this disclosure can effectively distinguish endometrial cancer from non-cancer individuals in tissue/urine/brush/swab samples. A single marker already achieves good detection performance, and combining multiple markers can further enhance detection efficacy.

The above embodiments are preferred implementations of the present disclosure, but the implementations of the present disclosure are not limited to these embodiments. Any changes, modifications, substitutions, combinations, or simplifications made without departing from the spirit and principles of the present disclosure shall be considered equivalent replacement methods and are included within the protection scope of the present disclosure.

## Claims

1. A methylated gene marker for endometrial cancer detection, wherein the methylated gene marker specifically comprises at least one of the VSX1, SYT1, ZNF132, and VWC2 genes.

2. A methylated gene marker for endometrial cancer detection, wherein the methylated gene marker comprises a methylated nucleic acid sequence in at least one target region of at least one target gene among VSX1, VWC2, ZNF132, and SYT1, and wherein the target region is selected from at least one of the following methylation segments in at least one of the VSX1, VWC2, ZNF132, and SYT1 genes:
VSX1 gene: Chr20: 25058334-25058603; Chr20: 25061937-25062310or Chr20: 25061874-25062088; Chr20: 25062398-25062760 or Chr20: 25062376-25062484; Chr20: 25062681-25062788or Chr20: 25062655-25062739; Chr20: 25062736-25062940 or Chr20: 25062749-25062900;
SYT1 gene: Chr12: 79258369-79258486 or Chr12: 79258392- 79258498;
ZNF132 gene: Chr19: 58951204-58951524 or Chr19: 58951402-58951524; Chr19: 58951554-58951637; Chr19: 58951672-58952006 or Chr19: 58951724-58951825;
VWC2 gene: Chr7: 49812992-49813086; Chr7: 49813051-49813343 or Chr7: 49813066-49813161; Chr7: 49813455-49813811 or Chr7: 49813387-49813537; Chr7: 49814721-49815073 or Chr7: 49814765-49814936; Chr7: 49815144-49815236; Chr7: 49815290-49815635 or Chr7: 49815340-49815439.

3. A detection primer for endometrial cancer detection for detecting a methylation status of a gene methylation region of the marker according to claim 2, wherein a nucleotide sequence of the detection primer is at least one of the following:
VSX1 gene:
Chr20: 25058334-25058603 corresponds to detection primer SEQ ID NO:1-2;
Chr20: 25061937-25062310 corresponds to detection primer SEQ ID NO:4-5;
Chr20: 25061874-25062088 corresponds to detection primer SEQ ID NO:6-7;
Chr20: 25062398-25062760 corresponds to detection primer SEQ ID NO:9-10;
Chr20: 25062376-25062484 corresponds to detection primer SEQ ID NO:12-13;
Chr20: 25062681-25062788 corresponds to detection primer SEQ ID NO:15-16;
Chr20: 25062655-25062739 corresponds to detection primer SEQ ID NO:18-19;
Chr20: 25062736-25062940 corresponds to detection primer SEQ ID NO:21-22;
Chr20: 25062749-25062900 corresponds to detection primer SEQ ID NO:23-24;
SYT1 gene:
Chr12: 79258369-79258486 corresponds to detection primer SEQ ID NO:26-27;
Chr12: 79258392-79258498 corresponds to detection primer SEQ ID NO:29-30;
ZNF132 gene:
Chr19: 58951204-58951524 corresponds to detection primer SEQ ID NO:32, SEQ ID NO:34;
Chr19: 58951402-58951524 corresponds to detection primer SEQ ID NO:33, SEQ ID NO:34;
Chr19: 58951554-58951637 corresponds to detection primers SEQ ID NO:36-37;
Chr19: 58951672-58952006 corresponds to detection primers SEQ ID NO:39-40;
Chr19: 58951724-58951825 corresponds to detection primers SEQ ID NO:41-42;
VWC2 gene:
Chr7: 49812992-49813086 corresponds to detection primers SEQ ID NO:44-45;
Chr7: 49813051-49813343 corresponds to detection primers SEQ ID NO:47-48;
Chr7: 49813066-49813161 corresponds to detection primers SEQ ID NO: 49-50;
Chr7: 49813455-49813811 corresponds to detection primers SEQ ID NO:52-53;
Chr7: 49813387-49813537 corresponds to detection primers SEQ ID NO:54-55;
Chr7: 49814721-49815073 corresponds to detection primers SEQ ID NO:57-58;
Chr7: 49814765-49814936 corresponds to detection primers SEQ ID NO:59-60;
Chr7: 49815144-49815236 corresponds to detection primers SEQ ID NO:62-63;
Chr7: 49815290-49815635 corresponds to detection primers SEQ ID NO:65-66;
Chr7: 49815340-49815439 corresponds to detection primers SEQ ID NO:67-68.

4. A detection probe for early screening and diagnosis of endometrial cancer for correspondingly detecting a methylation status of a gene methylation region of the marker according to claim 2, wherein a nucleotide sequence of the probe is at least one of the following:
VSX1 gene:
Chr20: 25058334-25058603 corresponds to detection probe SEQ ID NO:3;
Chr20: 25061937-25062310 corresponds to detection probe SEQ ID NO:8;
Chr20: 25061874-25062088 corresponds to detection probe SEQ ID NO:8;
Chr20: 25062398-25062760 corresponds to detection probe SEQ ID NO:11;
Chr20: 25062376-25062484 corresponds to detection probe SEQ ID NO:14;
Chr20: 25062681-25062788 corresponds to detection probe SEQ ID NO:17;
Chr20: 25062655-25062739 corresponds to detection probe SEQ ID NO:20;
Chr20: 25062736-25062940 corresponds to detection probe SEQ ID NO:25;
Chr20: 25062749-25062900 corresponds to detection probe SEQ ID NO:25;
SYT1 gene:
Chr12: 79258369-79258486 corresponds to detection probe SEQ ID NO:28;
Chr12: 79258392-79258498 corresponds to detection probe SEQ ID NO:31;
ZNF132 gene:
Chr19: 58951204-58951524 corresponds to detection probe SEQ ID NO:35;
Chr19: 58951402-58951524 corresponds to detection probe SEQ ID NO:35;
Chr19: 58951554-58951637 corresponds to detection probe SEQ ID NO:38;
Chr19: 58951672-58952006 corresponds to detection probe SEQ ID NO:43;
Chr19 : 58951724-58951825 corresponds to detection probe SEQ ID NO:43;
VWC2 gene:
Chr7: 49812992-49813086 corresponds to detection probe SEQ ID NO:46;
Chr7: 49813051-49813343 corresponds to detection probe SEQ ID NO:51;
Chr7: 49813066-49813161 corresponds to detection probe SEQ ID NO:51;
Chr7: 49813455-49813811 corresponds to detection probe SEQ ID NO:56;
Chr7: 49813387-49813537 corresponds to detection probe SEQ ID NO:56;
Chr7: 49814721-49815073 corresponds to detection probe SEQ ID NO:61;
Chr7: 49814765-49814936 corresponds to detection probe SEQ ID NO:61;
Chr7: 49815144-49815236 corresponds to detection probe SEQ ID NO:64;
Chr7: 49815290-49815635 corresponds to detection probe SEQ ID NO:69;
Chr7: 49815340-49815439 corresponds to detection probe SEQ ID NO:69.

5. Use of a reagent for detecting methylation of a target gene in preparation of a detection kit or device, wherein the detection kit or device is used for detecting, screening, or diagnosing endometrial cancer; and wherein the target gene is selected from at least one of the following genes: VSX1, SYT1, ZNF132, VWC2.

6. The use according to claim 5, wherein the reagent comprises at least one of an antibody, a probe a primer, and a mass spectrometry detection reagent specifically targeting the detection of the target gene; and wherein the primer comprises the detection primer according to claim 3.

7. A kit for diagnosing endometrial cancer, comprising a reagent for detecting methylation of a target gene, wherein the target gene is selected from at least one of the following genes: VSX1, SYT1, ZNF132, VWC2.

8. The kit according to claim 7, wherein the reagent comprises at least one of an antibody, a probe, a primer, and a mass spectrometry detection reagent specifically targeting the detection of the target gene; and wherein the primer comprises the detection primer according to claim 3; and the probe comprises the detection probe according to claim 4.

9. The kit according to claim 7 or 8, further comprising a primer pair and a probe for a reference gene, wherein the reference gene is an ACTB gene, and a nucleotide sequences of the primer pair for the reference gene ais as follows, and a nucleotide sequence of the probe is as follows:
Forward primer: 5'-TGGTGATGGAGGAGGTTTAGTAAGT-3'
Reverse primer: 5'- AACCAATAAAACCTACTCCTCCCTTAA -3'
Probe: 5'-ACCACCACCCAACACACAATAACAAACACA-3'.

10. Use of the methylated gene marker according to any one of claims 1-2, or the detection primer according to claim 3, or the detection probe according to claim 4, or the kit according to claims 8-9, in preparation of a product for diagnosing endometrial cancer.
